# EUROPEAN PATENT APPLICATION

(11) **EP 2 783 663 A1**
(43) Date of publication of application: **01.10.2014**
(21) Application number: 14161803.3
(22) Date of filing: 26.03.2014
(51) Int. Cl.: A61F 7/00, A61F 7/02

(54) **Thermoregulation device, thermoregulation system, and package**

(30) Priority: 26.03.2013 JP 2013063775; 20.02.2014 JP 2014030876
(71) Applicant: Nihon Kohden Corporation, Shinjuku-ku Tokyo (JP)
(72) Inventor: Hyogo, Mitsushi, Shinjuku-ku, Tokyo (JP); Shimada, Ichiro, Shinjuku-ku, Tokyo (JP); Tanaka, Eiichi, Shinjuku-ku, Tokyo (JP); Takayanagi, Yoshihiro, Shinjuku-ku, Tokyo (JP); Echigo, Masahiro, Shinjuku-ku, Tokyo (JP); Omote, Yuichiro, Osaka-shi, Osaka 530-8230 (JP); Yokoya, Kenji, Osaka-shi, Osaka 530-8230 (JP)
(74) Representative: Grünecker, Kinkeldey, Stockmair & Schwanhäusser

(57) **Abstract**

A thermoregulation device which is adapted to be used for covering part of a living body, includes: a first sheet which is higher in thermal conductivity than cotton cloth; a second sheet which is higher in heat insulating and retaining properties than cotton cloth; and a gas passage which is formed between the first sheet and the second sheet. A first opening which communicates with the gas passage is formed in one of the first sheet and the second sheet. A second opening which communicates with the gas passage is formed in one of the first sheet and the second sheet. A gas which is introduced through the first opening is discharged from the second opening via the gas passage.

## Description

### BACKGROUND

The present invention relates to a thermoregulation device which is used for covering part of a living body, a system which regulates the body temperature of the living body, and in which the thermoregulation device is used, and a package in which the thermoregulation device is packed in an airtight state.

Particularly in a medical facility, as a thermoregulation device of this type, a blanket which covers part of the body of a patient is used. As a blanket of this kind, there is a blanket in which a flow path through which liquid flows is formed in a sheet for covering the body (for example, see JP-T-2005-532141). When the temperature of the liquid is adequately controlled, heat is transferred between the liquid and the body of the patient through the sheet, thereby regulating the body temperature of the patient.

Liquid has a relatively low thermal conductivity. When the temperature of liquid is to be raised or lowered to a predetermined value, therefore, a larger amount of energy is necessary. Since liquid has a large weight per unit volume, the weight of a sheet in which a flow path is formed is increased, and a large burden is imposed on the patient who is covered with the sheet. Moreover, a countermeasure for preventing the liquid from leaking must be taken, and therefore it is difficult to suppress the production cost. Furthermore, maintenance such as replenishment and replacement of the liquid is required, and therefore the work efficiency is poor.

There is a related-art technique in which the body is cooled or warmed by using cloth having a structure through which cool air or warm air is passed. In this technique, in order to rapidly control the body temperature, however, it is necessary to increase the amount of the cool air or warm air which is flown to the skin surface of the patient. Therefore, the skin surface may be dried. In a configuration where a heating wire is used, such as an electric blanket, induced electrical noises may affect medical equipment connected to the patient.

On the other hand, there are related-art techniques for directly cooling the blood, such as that in which a cooling catheter is inserted into the vein, and that in which the blood is extracted to the outside of the body, and the extracted blood is cooled while being passed through cold water, and then returned into the body. In these techniques, although the body temperature can be rapidly controlled, invasion to the patient inevitably occurs.

### SUMMARY

The invention may provide a technique in which, while reducing the burden imposed on a patient, the body temperature can be efficiently regulated, and the production cost can be suppressed.

According to an aspect of the invention, there is provided a thermoregulation device which is adapted to be used for covering part of a living body, the thermoregulation device comprising: a first sheet which is higher in thermal conductivity than cotton cloth; a second sheet which is higher in heat insulating and retaining properties than cotton cloth; and a gas passage which is formed between the first sheet and the second sheet, wherein a first opening which communicates with the gas passage is formed in one of the first sheet and the second sheet, a second opening which communicates with the gas passage is formed in one of the first sheet and the second sheet, and a gas which is introduced through the first opening is discharged from the second opening via the gas passage. A porous material may be accommodated in the gas passage. The porous material may be detachably disposed.

At least the first sheet may be in a moist state or a gel state.

A pocket for accommodating a temperature regulating material may be formed at a position of the second sheet which is opposed to the gas passage.

The gas passage may be compartmentally formed by partly adhering the first sheet and the second sheet to each other.

The first sheet may be made of fiber cloth, and the second sheet may be a sheet in which a metal thin film is vapor-deposited on a surface that is opposed to the first sheet of the fiber cloth.

The thermoregulation device may be provided as a blanket that is adapted to overlay the living body to cover the part of the living body, or that is adapted to be laid under the living body.

The thermoregulation device may be provided as an orthosis that is adapted to be attached to the living body to cover the part of the living body, and that includes a holding member adapted to maintain a state where the orthosis is attached to the living body.

According to an aspect of the invention, there is also provided a thermoregulation system which is configured to regulate a body temperature of a living body, the thermoregulation system comprising: a thermoregulation device which is adapted to be used for covering part of the living body; and a gas supply apparatus which is configured to supply a temperature-controlled gas, wherein the thermoregulation device includes: a first sheet which is higher in thermal conductivity than cotton cloth; a second sheet which is higher in heat insulating and retaining properties than cotton cloth; and a gas passage which is formed between the first sheet and the second sheet, a first opening which communicates with the gas passage is formed in one of the first sheet and the second sheet, a second opening which communicates with the gas passages is formed in one of the first sheet and the second sheet, and the temperature-controlled gas is introduced through the first opening, and discharged from the second opening via the gas passage.

The thermoregulation system may further include a gas discharge apparatus which is connected to the second opening, and which is configured to forcibly discharge the temperature-controlled gas in the gas passage.

The gas supply apparatus and the gas discharge apparatus may form a gas circulation path which is configured to again send the temperature-controlled gas discharged from the second opening, to the first opening.

According to an aspect of the invention, there is also provided a package comprising: a thermoregulation device which is adapted to be used for covering part of a living body; and a package bag in which the thermoregulation device is packed in an airtight state, wherein the thermoregulation device includes: a first sheet which is higher in thermal conductivity than cotton cloth; a second sheet which is higher in heat insulating and retaining properties than cotton cloth; and a gas passage which is formed between the first sheet and the second sheet, a first opening which communicates with the gas passage is formed in one of the first sheet and the second sheet, a second opening which communicates with the gas passage is formed in one of the first sheet and the second sheet, and at least the first sheet is in a moist state or a gel state.

The thermoregulation device may be accommodated in the package bag in a folded state.

### BRIEF DESCRIPTION OF THE DRAWINGS

Fig. 1 is a view diagrammatically showing the manner of using blankets according to the invention.
Figs. 2A and 2B are top and bottom views showing the configuration of a cover blanket.
Fig. 3 is a sectional view showing the internal configuration of the cover blanket.
fig. 4 is a sectional view diagrammatically showing the configuration of a second sheet in the blanket.
Fig. 5 is a sectional view diagrammatically showing a state where the blanket is packed in an airtight package.
Figs. 6A and 6B are bottom and sectional views showing the configuration of a bottom blanket.
Figs. 7A and 7B are top and sectional views showing the configuration of a cover blanket of a modification.
Figs. 8A to 8C are views diagrammatically showing the manner of using orthoses of a modification.

### DETAILED DESCRIPTION OF EXEMPLARY EMBODIMENTS

Hereinafter, an embodiment of the invention will be described in detail with reference to the accompanying drawings. In the drawings which will be used in the following description, the scale is adequately changed in order to draw components in a recognizable size.

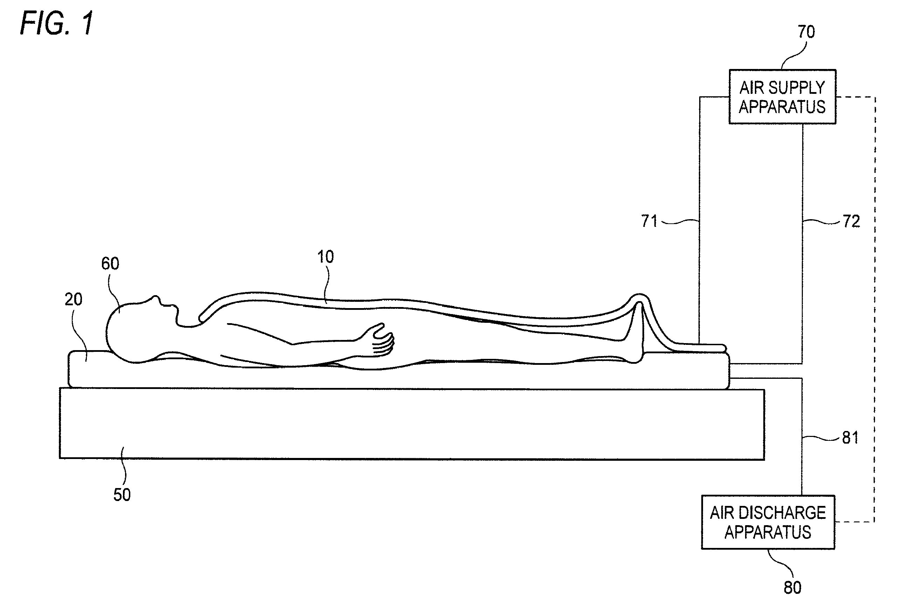

Fig. 1 is a view diagrammatically showing the manner of using a cover blanket 10 and bottom blanket 20 (an example of the thermoregulation device) of an embodiment of the invention. These blankets are used for controlling hypothermia and rewarming in the hypothermia therapy which is performed in, particularly, a medical facility. The blankets are used for preventing the body temperature of a patient before, during, or after surgery, or during treatment in an intensive care unit or a general ward, from lowering or rising.

The bottom blanket 20 is spread on a bed 50, and a patient 60 or the living body lies down thereon. The patient 60 is covered by the cover blanket 10. Therefore, a state is attained where the cover blanket 10 covers part of the body of the patient 60, and the bottom blanket 20 covers part of the back side of the patient 60.

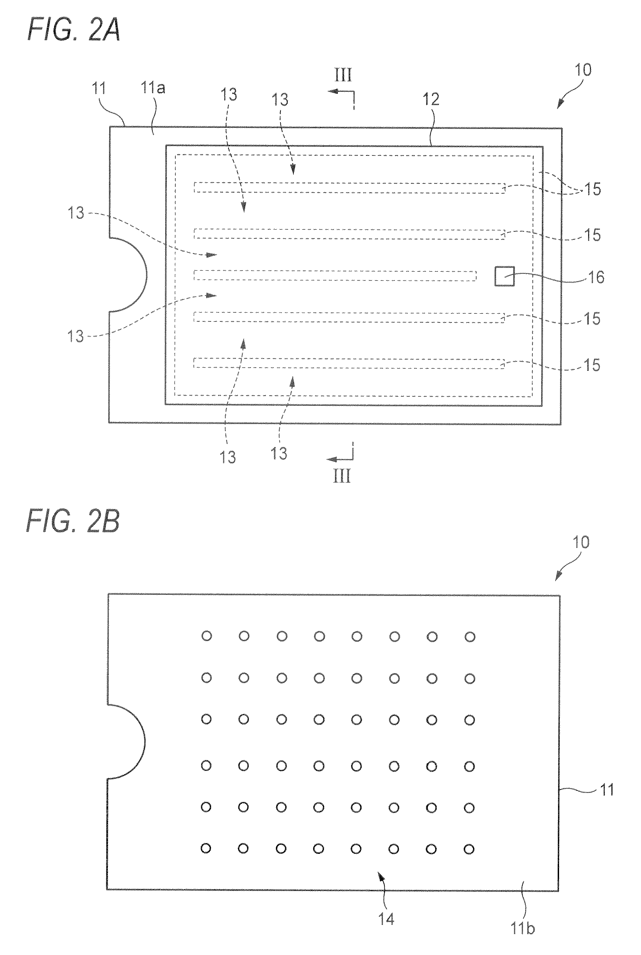

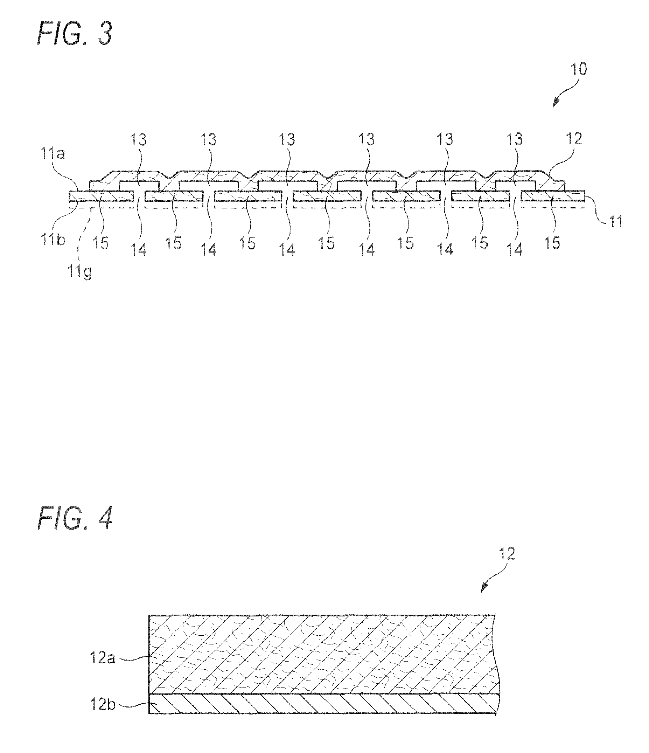

Figs. 2A and 2B are views showing the configuration of the cover blanket 10, Fig. 2A is a top view, and Fig. 2B is a bottom view. Fig. 3 is a sectional view taken along line III-III in Fig. 2A. As shown in these figures, the cover blanket 10 of the embodiment has a configuration where a second sheet 12 is partly adhered to a first sheet 11 to compartmentally form gas passages 13 therebetween.

In order to efficiently transmit the energy of warm or cool air flowing through the gas passages 13 toward the patient 60, the first sheet 11 is configured by fiber cloth having a thermal conductivity which is higher than that of cotton cloth. Specifically, the sheet is formed by a usual method into non-woven cloth or woven/knitted cloth by using synthetic fibers such as polyester fibers, polyamide fibers, polyolefin fibers, or regenerated cellulose fibers. Preferably, the fibers have the filament structure.

Examples of polyester fibers are polyethyleneterephthalate, polytrimethyleneterephthalate, and polybutyleneterephthalate. Examples of polyamide fibers are nylon 6 and nylon 66. Examples of polyolefin fibers are polypropylene and polyethylene. Examples of regenerated cellulose fibers are cuprammonium rayon, viscose rayon (including high-tenacity rayon, polynosic rayon, and high wet modulus rayon), and purified cellulose (lyocell).

With respect to an index of the thermal conductivity, preferably, the index of contact warm/cool feeling (q-max) is 0.20 W/cm² or more in an evaluation method using a thermal property measuring apparatus (for example, Thermo Lab II manufactured by Katoh tech Co., Ltd.). More preferably, the index is 0.25 W/cm² or more.

In order to efficiently transmit the energy, preferably, the first sheet 11 is thin and has a smooth surface. Therefore, it is preferable that the thickness is 0.30 mm or less and the basis weight is 90 g/cm² or less. More preferably, the thickness is 0.20 mm or less and the basis weight is 60 g/cm² or less. When a high-density knitted cloth is prepared by using a polyamide fiber having a thickness of 56 dtex or less, particularly, it is possible to obtain a sheet which is flat, smooth, thin, and light in weight, and which has an excellent packaging property. In order to make the surface flat and smooth, a hot roll calender process, an emboss process, resin coating, or the like may be applied.

In order to efficiently transmit the energy, furthermore, it is preferable that the air permeability of the sheet is high. In the embodiment, as shown in Fig. 2B, many ventilation holes 14 are formed in the first sheet 11, thereby enhancing the air permeability. By contrast, when the air permeability is excessively high, the air circulation in the blanket is impaired, and the energy loss is increased. Therefore, preferably, the air permeability of the first sheet 11 is 10.0 cm³/cm²·sec or less in an evaluation according to JIS-L-1096 Method A (Fragile method). More preferably, the air permeability is 5.0 cm³/cm²·sec or less.

In order to prevent the energy of the warm or cool air flowing through the gas passages 13 from escaping to the outside, the second sheet 12 includes fiber cloth 12a having heat insulating and retaining properties which are higher than those of cotton cloth. Specifically, the sheet is formed by a usual method into non-woven cloth or woven/knitted cloth by using fibers such as polyester fibers, polyolefin fibers, acrylic fibers, PPS (PolyPhenylene Sulfide) fiber, or wool.

Examples of polyester fibers are polyethylene terephthalate, polytrimethylene terephthalate, and polybutylene terephthalate. Examples of polyolefin fibers are polypropylene and polyethylene. In the case where polyester or polypropylene is used, particularly, a sheet having an excellent heat insulating property can be produced at a relatively low cost.

With respect to an index of the heat retaining property, preferably, the heat retention rate is 10% or more in an evaluation method using a thermal property measuring apparatus measurement (for example, Thermo Lab II manufactured by Katoh tech Co., Ltd.). More preferably, the rate is 15% or more.

In order to enhance the heat retaining retention property, it is preferable to thicken the second sheet 12. When the thickness is made excessively large, however, the packaging property is lowered, the weight is increased, and the circulation of the air flow is impaired. Therefore, it is preferable that the thickness is 0.50 mm or less and the basis weight is 100 g/cm² or less. More preferably, the thickness is 0.30 mm or less and the basis weight is 50 g/cm² or less.

In order to enhance the heat insulating property, it is preferable to employ a structure having many layers of air. In order to attain such a structure, a crimping process, a napping process, a flocking process, or the like may be applied to fibers.

In order to prevent the warm or cool air flowing through the gas passages 13 from escaping to the outside, the second sheet 12 has a low air permeability. In order to reduce the air permeability, a hot roll calender process, an emboss process, resin coating, a laminating process, or the like may be applied to the sheet. Preferably, the air permeability of the second sheet 12 is 0.5 cm³/cm²·sec or less in an evaluation according to JIS-L-1096 Method A (Fragile method). More preferably, the air permeability is 0.2 cm³/cm²·sec or less.

In the second sheet 12, a metal layer may be formed for the purpose of heat insulation and prevention of heat transfer due to infrared radiation. In the embodiment, as shown in Fig. 4, the second sheet 12 has a configuration where an aluminum thin film 12b which is an example of a metal thin film is vapor-deposited on one surface of the fiber cloth 12a. Other examples of the metal thin film are stainless steel, titanium, gold, silver, copper, tin, platinum, chromium, nickel, and alloys of these metals. Preferably, a metal having a reflectivity of 93% or more with respect to infrared rays having a wavelength of 1. 0 µm may be used. From the viewpoints of the cost, the safety, the easy processing, or the like, titanium, aluminum, and alloys of these metals and another metal are preferable.

Examples of the method of forming the metal thin film are foil printing, metal coating, electroless plating, sputtering, and vacuum vapor deposition. In order to attain a desired heat insulating property without impairing the texture, it is requested to form a thin and uniform film. From this point of view, sputtering and vacuum vapor deposition are preferable.

Both the first sheet 11 and the second sheet 12 are formed to include fiber cloth. Therefore, the cover blanket 10 can be configured so as to be excellent in flexibility and shape restoring property.

The first sheet 11 and the second sheet 12 are bonded to each other in a state where the surface on which the aluminum thin film 12b is formed is opposed to the upper surface 11a of the first sheet 11. The bonding is performed by a related-art method through adhesive portions 15 which are indicated by the broken lines in Fig. 2A. In both the first sheet 11 and the second sheet 12, a thermoplastic polymer is used as the main material (or coating of a thermoplastic polymer is applied). Therefore, for example, the first sheet 11 and the second sheet 12 can be easily heat-adhered to each other, and a complicated structure may be formed.

In the second sheet 12, an air introduction port 16 which is the first opening in the invention is formed. The air introduction port 16, and the ventilation holes 14 which are formed in the first sheet 11, and which function as the second opening in the invention communicate with the gas passages 13 which are formed between the first sheet 11 and the second sheet 12 as a result of the above-described bonding.

The air introduction port 16 is a portion to which an air supply tube 71 extending from an air supply apparatus 70 shown in Fig. 1 is connected. The air supply apparatus 70 has a related-art configuration which can supply a predetermined flow rate of air that is controlled to a given temperature. The air which is sent from the air supply apparatus 70 to the air introduction port 16 through the air supply tube 71 flows through the gas passages 13, and then is discharged from the ventilation holes 14 which are opened in the lower surface 11b of the first sheet 11.

The cover blanket 10 is used while the lower surface 11b of the first sheet 11 is directed toward the side which is in contact with the patient 60. The temperature-controlled air which is supplied from the air supply apparatus 70 to the cover blanket 10 is ejected toward the patient 60 from the ventilation holes 14 which are opened in the lower surface 11b.

The first sheet 11 which is in contact with the patient 60 has a relatively high thermal conductivity, and the aluminum thin film 12b of the second sheet 12 which is placed so as to be opposed to the patient 60 prevents the temperature of the gas flowing through the gas passages 13 from escaping to the outside. Therefore, the body temperature of the patient 60 is enabled to efficiently reach a target value, and the controlled body temperature can be efficiently maintained.

Since air is used as a temperature controlling medium, the heat conduction efficiency can be improved as compared with the case where liquid is used. When comparing with the case where liquid is used, the weight in use of the cover blanket 10 can be remarkably reduced. Therefore, the ease of handling is improved, and the burden on the patient 60 due to the weight can be suppressed. Moreover, a configuration for preventing liquid from leaking is not necessary, and hence the production cost can be reduced.

Since the body temperature control can be efficiently performed, the amount of the air ejected from the ventilation holes 14 can be reduced to the required minimum. Therefore, the drying of the skin of the patient 60 due to direct impingement of the ejected air can be suppressed.

In the embodiment, the cover blanket 10 is used in a moist state which is obtained by previously immersing the blanket in water or volatile liquid such as alcohol. In the case where a control of lowering the body temperature is performed, when the liquid is vaporized, particularly, heat is absorbed from the environment, and therefore the body temperature is enabled to efficiently reach a target value. Moreover, the drying of the skin of the patient 60 due to the air ejected from the ventilation holes 14 can be prevented from occurring.

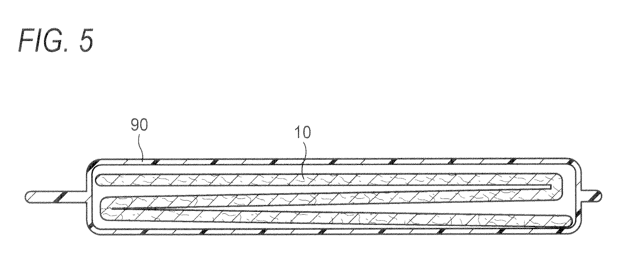

As shown in Fig. 5, the cover blanket 10 before use is accommodated in a package bag 90 in a state where the blanket is previously set to a moist state and folded. The package bag 90 is a related-art bag which is made of a film such as vinyl chloride, polyethylene, or polypropylene. When the inlet of the bag is heat welded after the cover blanket 10 is accommodated, the cover blanket 10 is packed in an airtight package. According to the configuration, while maintaining the state where the temperature control is enabled to be efficiently performed by evaporation of water or liquid, the blanket can be set in the course of distribution, and in storage. The cover blanket 10 after use is discarded.

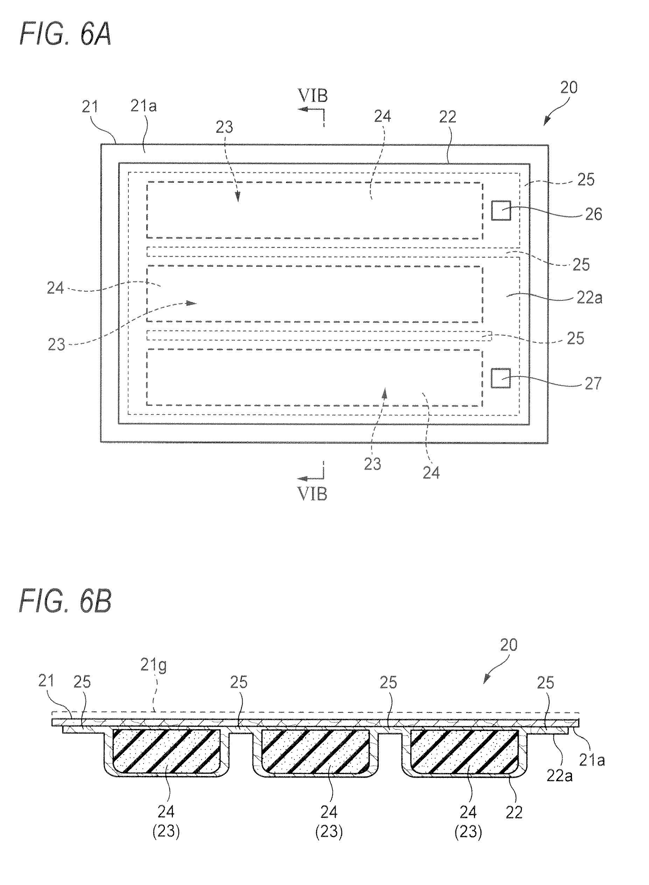

Next, the configuration of the bottom blanket 20 will be described with reference to Figs. 6A and 6B. Fig. 6A is a bottom view, and Fig. 6B is a sectional view taken along line VIB-VIB in Fig. 6A. The bottom blanket 20 of the embodiment has a configuration where a second sheet 22 is partly adhered to a first sheet 21 to compartmentally form gas passages 23 therebetween. The materials of the first and second sheets 21, 22 are similar to those of the first and second sheets 11, 12 of the cover blanket 10, respectively, and therefore repeated description is omitted.

The first sheet 21 and the second sheet 22 are bonded to each other in a state where the surface on which an aluminum thin film is formed is opposed to the lower surface 21a of the first sheet 21. The bonding is performed by a related-art method through adhesive portions 25 which are indicated by the broken lines in Fig. 6A.

In the lower surface 22a of the second sheet 22, an air introduction port 26 which is the first opening in the invention, and an air discharge port 27 which is the second opening in the invention are formed. The air introduction port 26 and the air discharge port 27 communicate with gas passages 23 which are formed between the first sheet 21 and the second sheet 22 as a result of the above-described bonding.

A porous material 24 is housed in each of the gas passages 23. Slits which are not shown are formed in the second sheet 22, and the porous materials 24 can be inserted and extracted through the slits.

In order to receive the weight of the patient 60 and ensure gas passages for warm or cool air, the porous materials 24 are requested to have an adequate cushioning property and a high air permeability. For example, a three-dimensional net-like structure may be employed where many continuous filamentous structures configured by a thermoplastic resin and having a diameter of 0.1 to 1.5 mm (preferably, 0.2 to 1.0 mm) are caused to meander in a loop-like manner, and mutual contact portions are fuse bonded to one another. Examples of the thermoplastic resin are thermoplastic elastomer resins including thermoplastic polyester elastomer and thermoplastic urethane elastomer.

From the viewpoint that, when the weight of the patient 60 is received, sufficient gas passages are ensured and the ease of handling is assured, preferably, the porous materials 24 have a thickness of from 10 mm or more to 50 mm or less. More specifically, the thickness is from 15 mm or more to 50 mm or less. Preferably, the density of the porous materials 24 is from 24 kg/m³ or more to 200 kg/m³ or less. More preferably, the density is 30 kg/m³ or more, and further preferably 40 kg/m³ or more.

From the viewpoint that gas passages for warm or cool air are ensured and energy is sufficiently transferred, preferably, the air permeability of the porous materials 24 is 300 cm³/cm²·sec or more in an evaluation according to JIS-L-1096 Method A (Fragile method). More preferably, the air permeability is 400 cm³/cm²·sec or more.

The air introduction port 26 is a portion to which an air supply tube 72 extending from the air supply apparatus 70 shown in Fig. 1 is connected. The air discharge port 27 is a portion to which an air discharge tube 81 extending from an air discharge apparatus 80 shown in Fig. 1 is connected. The air discharge apparatus 80 has a related-art configuration which can suck air at a predetermined flow rate.

The air which is sent from the air supply apparatus 70 to the air introduction port 26 through the air supply tube 72 flows through the gas passages 23, and then is discharged from the air discharge port 27 which is opened in the lower surface 22a of the second sheet 22. The air discharge is forcibly performed through the air discharge port 27 by the suction operation of the air discharge apparatus 80. Also in the case where the porous materials 24 are placed in the gas passages 23, therefore, the air flow is smoothened.

The bottom blanket 20 is used while the upper surface 21b of the first sheet 21 is directed toward the side which is in contact with the patient 60. In the case where the patient 60 lies down on the bottom blanket 20 as shown in Fig. 1, although the porous materials 24 are partly deformed by the weight of the patient 60, the whole gas passages 23 are not collapsed and clogged. Therefore, the temperature controlled air can be smoothly passed also on the back side of the patient 60.

The first sheet 21 which is in contact with the patient 60 has a relatively high thermal conductivity, and the aluminum thin film of the second sheet 22 which is placed so as to be opposed to the patient 60 prevents the temperature of the gas flowing through the gas passages 123 from escaping to the outside. Therefore, the body temperature of the patient 60 is enabled to efficiently reach a target value, and the controlled body temperature can be efficiently maintained.

Since air is used as a temperature controlling medium, the heat conduction efficiency can be improved as compared with the case where liquid is used. Moreover, a configuration for preventing liquid from leaking is not necessary, and hence the production cost can be reduced.

In the embodiment, the bottom blanket 20 is used in a moist state which is obtained by previously immersing the blanket in water or volatile liquid such as alcohol. In the case where a control of lowering the body temperature is performed, when water or liquid is vaporized, particularly, the body temperature is enabled to efficiently reach a target value. Moreover, also heat of the gas flowing through the gas passages 23 is absorbed. In the case of a configuration (indicated by the broken line in Fig. 1) where the gas discharged from the air discharge port 27 is recovered and recirculated through the air introduction port 26, therefore, it is possible to suppress the energy which is required to lower the temperature to a preset temperature because the temperature of the gas which is recirculated to the air introduction port 26 is lower than the room temperature.

Similarly with the cover blanket 10, the bottom blanket 20 before use is accommodated in the package bag 90 shown in Fig. 5 in a state where the blanket is previously set to a moist state and folded. The user takes out the bottom blanket 20 from the package bag 90 and spreads the bottom blanket 20. Then, the user inserts the porous materials 24 into the gas passages 23 through the slits formed in the second sheet 22, to set the bottom blanket 20 to a usable state. The bottom blanket 20 after use is discarded. The porous materials 24 may be discarded, or, when necessary sterilization treatment is performed, can be reused.

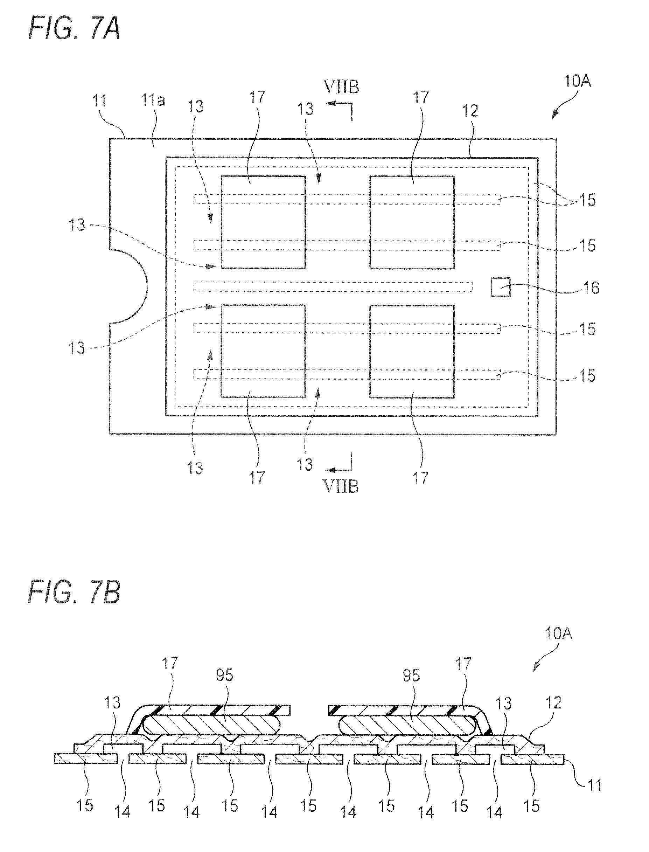

Next, the configuration of a cover blanket 10A of a modification will be described with reference to Figs. 7A and 7B. Fig. 7A is a top view, and Fig. 7B is a sectional view taken along line VIIB-VIIB in Fig. 7A. The cover blanket 10A is different from the cover blanket 10 in the above-described embodiment in that pockets 17 for accommodating temperature regulating materials 95 are disposed on the outer surface of the second sheet 12.

An appropriate number of pockets 17 are disposed at positions opposed to the gas passages 13. The temperature regulating materials 95 are related-art exothermic or coolant materials. When the temperature regulating materials 95 are inserted into the pockets 17, generated warm or cool air is carried by air flowing through the gas passages 13, and the body temperature control can be efficiently performed. Moreover, the temperature regulating materials 95 function as an adequate weight, and therefore the contact property between the first sheet 11 and the patient 60 is increased. Therefore, the efficiency of the body temperature control can be improved.

The embodiment has been described in order to facilitate understanding of the invention, and is not intended to limit the invention. It is a matter of course that the invention may be changed or improved without departing the spirit thereof, and includes equivalents thereof.

The shape and size of the thermoregulation device are not limited to those above described. The shape and the size can be adequately changed in accordance with the portion of the patient 60 which must be subjected to the body temperature regulation, and the purposes of use and treatment.

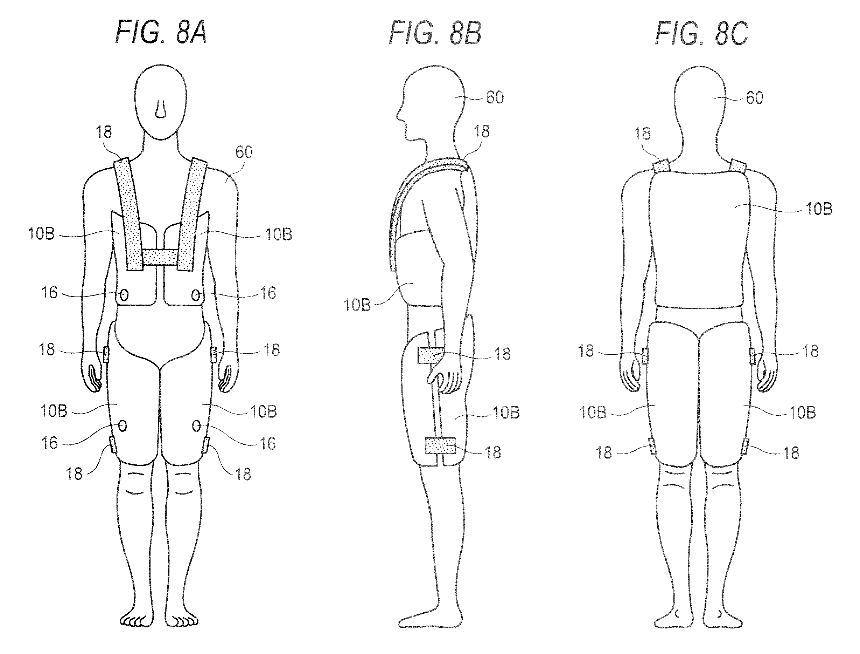

As shown in Figs. 8A to 8C, for example, the thermoregulation device may have configurations of orthoses 10B. The orthoses 10B may have a shape which allows the orthoses to be attached to the patient 60 so as to surround part of the body. The structures of the orthoses 10B themselves are similar to the structure of the above-described cover blanket 10, and therefore repeated description is omitted. Fig. 8A shows the patient 60 as viewed from the front side, Fig. 8B shows the patient 60 as viewed from the right side, and Fig. 8C shows the patient 60 as viewed from the rear side. In this example, the orthoses 10B are attached to the patient 60 so as to respectively surround the torso, the right thigh, and the left thigh. Alternatively, the orthoses may be attached so as to surround the arms or the legs. In the alternative, the orthoses 10B have a shape appropriate for attachment to the arms or the legs. The above-described air introduction port 16 is disposed in each of the orthoses 10B.

Each of the orthoses 10B includes holding members 18. The shape and arrangement of the holding members 18 are set so that, even in a state where the patient 60 stands up, the members can maintain the attached state of the orthosis 10B. For example, one end portions of the holding members 18 are fixed to parts of the orthosis 10B, and the other end portions of the holding members 18 are detachably attached to other parts of the orthosis 10B. The configuration for attaining the detachable attachment may use a snap fit, a hook and loop fastener, or the like. The holding members 18 may be disposed so as to couple independent orthoses 10B with each other.

According to the configuration, even when the patient 60 is not lying on the bed 50, it is possible to continue the hypothermia therapy. Therefore, the degree of freedom of the patient 60 during treatment is enhanced.

The configuration of the second sheets 12, 22 is not limited to that where an aluminum thin film is vapor-deposited on one surface of cloth. Alternatively, a configuration where cloth is formed by using fibers on which aluminum is vapor-deposited may be employed. The vapor deposition may be performed by using a metal other than aluminum as far as the metal has a desired ability of reflecting infrared rays.

The shape and arrangement of the gas passages 13, 23 are not limited to those above described. The shape and the size can be adequately changed in accordance with the portion of the patient 60 which must be subjected to the body temperature regulation, and the purposes of use and treatment, as far as the gas passages are formed between the first sheet 11 or 21 and the second sheet 12 or 22.

In the embodiment, mainly from the viewpoint of suppression of the production cost, the gas passages 13, 23 are compartmentally formed by partly adhering the first sheet 11 or 21 and the second sheet 12 or 22 to each other. However, the gas passages 13, 23 may be compartmentally formed by various methods as far as the gas passages are formed between the first sheet 11 or 21 and the second sheet 12 or 22. For example, a configuration where the gas passages 13, 23 are compartmentally formed by placing partition members at adequate positions may be employed.

The air introduction port 16 of the cover blanket 10 (10A) may be disposed in the first sheet 11 in place of the second sheet 12. The air introduction port 26 and air discharge port 27 of the bottom blanket 20 may be disposed in the first sheet 21 in place of the second sheet 22.

The air discharge apparatus 80 is not always required to be connected to the air discharge port 27 of the bottom blanket 20. When the air flow in the gas passages 23 is sufficiently ensured by, for example, increasing the flow rate of air supplied from the air supply apparatus 70, a configuration where air is spontaneously discharged through the air discharge port 27 may be employed.

In place of formation of the ventilation holes 14 in the cover blanket 10 (10A), an air discharge port which communicates with the gas passages 13, and which is connected to the air discharge apparatus 80 as required may be disposed. In place of or in addition to disposition of the air discharge port 27 in the bottom blanket 20, a ventilation hole which communicates with the gas passages 23, and from which air is ejected may be formed.

At least the surfaces of the first sheets 11, 21 which are in contact with the patient 60 may be in a gel state. Specifically, as indicated by the broken lines in Figs. 3 and 6B, polymer gel layer 11g, 21g may be formed on the surfaces of the first sheets 11, 21, respectively. When the water content contained in the gel is vaporized, heat is absorbed from the environment, whereby the cooling effect is promoted. In this case, when adhesive gel which is widely used in the field of a biological signal measuring electrode, the contact areas between the first sheets 11, 21 and the patient 60 are increased, and the temperature control efficiency can be improved.

In the cover blanket 10 (10A), the bottom blanket 20, and the orthoses 10B, it is not always required that the whole is set to a moist or gel state. It is required only that at least the first sheets 11, 21 which are in contact with the patient 60 are set to a moist or gel state. In the case where the blankets are used only for warming, the sheets are not necessary to be previously set to a moist or gel state.

With respect to the size of the package bag 90, when there is no problem in transportation and storage, the cover blanket 10 (10A), the bottom blanket 20, and the orthoses 10B are not always required to be airtightly packaged in a folded state.

According to the configurations of the thermoregulation device and the thermoregulation system related to an aspect of the invention, the first sheet having a relatively high thermal conductivity is used while being directed toward the side which is in contact with the living body, whereby the temperature of the gas passing through the gas passage can be efficiently transmitted to the living body. By contrast, the second sheet has relatively high heat insulating and retaining properties, and hence prevents the energy of the gas passing through the gas passage, from escaping to the outside. Therefore, it is possible to cause the body temperature of the living body to rapidly reach a target value without causing invasion. Moreover, the controlled body temperature can be efficiently maintained.

Since the gas is used as a temperature controlling medium, the heat conduction efficiency can be improved as compared with the case where liquid is used. When comparing with the case where liquid is used, the weight in use of the thermoregulation device can be remarkably reduced. Therefore, the ease of handling is improved, and the burden imposed on the living body due to the weight can be suppressed. Moreover, a configuration for preventing liquid from leaking is not necessary, and hence the production cost can be reduced. Furthermore, maintenance such as replenishment and replacement of the liquid is not required.

A porous material may be accommodated in the gas passage. In this case, the thermoregulation device can be laid under the living body. The porous material receives the weight the living body. Therefore, a situation where the gas passage is collapsed and clogged does not occur, and the temperature controlled gas can flow beneath the living body.

The porous material may be detachably disposed. In this case, the porous material is requested to be inserted into the gas passage only when the thermoregulation device is to be used. Therefore, the thermoregulation device can be easily handled before and after the use. Moreover, the porous material can be reused.

At least the first sheet may be in a moist state or a gel state. In the case where the temperature reduction control is performed, particularly, water or liquid which causes the moist or gel state is vaporized to absorb heat from the environment, whereby the body temperature is enabled to efficiently reach a target value. Also heat of the gas passing through the gas passage is absorbed. In the case of a configuration where the gas discharged from the second opening is recovered and recirculated through the first opening, therefore, it is possible to suppress the energy which is required to lower the temperature of the gas supplied to the first opening to a preset temperature. In the case where the gas discharged from the second opening is blown to the living body, the drying of the skin of the living body due to the gas can be suppressed.

In a configuration where the gel has adhesiveness, the contact area between the first sheet and the living body is increased, and the temperature control efficiency can be improved.

A pocket for accommodating a temperature regulating material may be formed at a position of the second sheet which is opposed to the gas passage. In this case, hot air or cold air produced by the temperature regulating material inserted into the pocket is carried by the gas flowing through the gas passage, and the body temperature control can be efficiently performed. In the case where the thermoregulation device is used as a cover sheet, the temperature regulating material functions as an adequate weight, and therefore the contact property between the first sheet and the living property is enhanced. Therefore, the efficiency of the body temperature control can be improved.

The gas passage may be compartmentally formed by partly adhering the first sheet and the second sheet to each other. In this case, the desired gas passage can be easily formed in a partitioned manner while suppressing the production cost.

The first sheet may be made of fiber cloth, and the second sheet may be a sheet in which a metal thin film is vapor-deposited on a surface that is opposed to the first sheet of the fiber cloth. The use of the fiber cloth allows the thermoregulation device which is excellent in flexibility and shape restoring property, to be provided. Moreover, since the metal thin film reflects heat radiated from the gas flowing through the gas passage and the outside air, the heat insulating effect is enhanced. Therefore, the temperature control for the gas flowing through the gas passage can be efficiently performed.

The thermoregulation system may further include a gas discharge apparatus which is connected to the second opening, and which is configured to forcibly discharge the temperature-controlled gas in the gas passage. In this case, the gas can smoothly flows through the gas passage.

The gas supply apparatus and the gas discharge apparatus may form a gas circulation path which is configured to again send the temperature-controlled gas discharged from the second opening, to the first opening. In this case, it is possible to suppress the energy which is required to control the temperature in the gas supply apparatus.

According to the configuration of the package related to an aspect of the invention, while maintaining the state where the temperature control is enabled to be efficiently performed by evaporation of water or liquid, the thermoregulation device can be set in the course of distribution, and in storage. Moreover, the thermoregulation device can be used even immediately after opening the package bag.

The thermoregulation device may be accommodated in the package bag in a folded state. In this case, the size of the package can be reduced. Therefore, the easiness of handling in transportation and storage is improved.

The thermoregulation device may be realized in the form of an orthosis in place of the forms of the above-described cover and bottom sheets (blankets). The thermoregulation device has a shape which is to be attached to the living body so as to surround part of the living body. In this case, preferably, the thermoregulation device may include a holding member which maintains the attached state.

According to the configuration, even when the patient is not lying on a bed, it is possible to continue the hypothermia therapy. Therefore, the degree of freedom of the patient during treatment is enhanced.

## Claims

1. A thermoregulation device which is adapted to be used for covering part of a living body, the thermoregulation device comprising:
a first sheet which is higher in thermal conductivity than cotton cloth;
a second sheet which is higher in heat insulating and retaining properties than cotton cloth; and
a gas passage which is formed between the first sheet and the second sheet, wherein
a first opening which communicates with the gas passage is formed in one of the first sheet and the second sheet,
a second opening which communicates with the gas passage is formed in one of the first sheet and the second sheet, and
a gas which is introduced through the first opening is discharged from the second opening via the gas passage.

2. The thermoregulation device according to claim 1, wherein a porous material is accommodated in the gas passage.

3. The thermoregulation device according to claim 2, wherein the porous material is detachably disposed.

4. The thermoregulation device according to any one of claims 1 to 3, wherein at least the first sheet is in a moist state or a gel state.

5. The thermoregulation device according to any one of claims 1 to 4, wherein a pocket for accommodating a temperature regulating material is formed at a position of the second sheet which is opposed to the gas passage.

6. The thermoregulation device according to any one of claims 1 to 5, wherein the gas passage is compartmentally formed by partly adhering the first sheet and the second sheet to each other.

7. The thermoregulation device according to any one of claims 1 to 6, wherein
the first sheet is made of fiber cloth, and
the second sheet is a sheet in which a metal thin film is vapor-deposited on a surface that is opposed to the first sheet of the fiber cloth.

8. The thermoregulation device according to any one of claims 1 to 7, which is provided as a blanket that is adapted to overlay the living body to cover the part of the living body, or that is adapted to be laid under the living body.

9. The thermoregulation device according to any one of claims 1 to 7, which is provided as an orthosis that is adapted to be attached to the living body to cover the part of the living body, and that includes a holding member adapted to maintain a state where the orthosis is attached to the living body.

10. A thermoregulation system which is configured to regulate a body temperature of a living body, the thermoregulation system comprising:
a thermoregulation device which is adapted to be used for covering part of the living body; and
a gas supply apparatus which is configured to supply a temperature-controlled gas, wherein
the thermoregulation device includes:
a first sheet which is higher in thermal conductivity than cotton cloth;
a second sheet which is higher in heat insulating and retaining properties than cotton cloth; and
a gas passage which is formed between the first sheet and the second sheet,
a first opening which communicates with the gas passage is formed in one of the first sheet and the second sheet,
a second opening which communicates with the gas passages is formed in one of the first sheet and the second sheet, and
the temperature-controlled gas is introduced through the first opening, and discharged from the second opening via the gas passage.

11. The thermoregulation system according to claim 10, further comprising a gas discharge apparatus which is connected to the second opening, and which is configured to forcibly discharge the temperature-controlled gas in the gas passage.

12. The thermoregulation system according to claim 11, wherein the gas supply apparatus and the gas discharge apparatus form a gas circulation path which is configured to again send the temperature-controlled gas discharged from the second opening, to the first opening.

13. A package comprising:
a thermoregulation device which is adapted to be used for covering part of a living body; and
a package bag in which the thermoregulation device is packed in an airtight state, wherein
the thermoregulation device includes:
a first sheet which is higher in thermal conductivity than cotton cloth;
a second sheet which is higher in heat insulating and retaining properties than cotton cloth; and
a gas passage which is formed between the first sheet and the second sheet,
a first opening which communicates with the gas passage is formed in one of the first sheet and the second sheet,
a second opening which communicates with the gas passage is formed in one of the first sheet and the second sheet, and
at least the first sheet is in a moist state or a gel state.

14. The package according to claim 13, wherein the thermoregulation device is accommodated in the package bag in a folded state.
